# EUROPEAN PATENT APPLICATION

(11) **EP 2 476 426 A1**
(43) Date of publication of application: **18.07.2012**
(21) Application number: 11000260.7
(22) Date of filing: 13.01.2011
(51) Int. Cl.: A61K 38/16, A61P 3/10

(54) **Treatment and prevention of symptoms of metabolic syndrome**

(71) Applicant: ETH Zurich, 8092 Zurich (CH)
(72) Inventor: Wolfrum, Christian, 8046 Zürich (CH); Meissburger, Bettina, 8050 Zürich (CH); Mrosek, Nadja, 5210 Windisch (CH)

(57) **Abstract**

The invention relates to the use of S100A4 (UniProtKB P26447) in the treatment and prevention of symptoms of metabolic syndrome, in particular in the treatment of insulin resistance and type 2 diabetes. This use is preferably indicated in pregnant or breastfeeding women, and in patients with severe heart disease, renal insufficiency and/or liver insufficiency.

## Description

### Field of the Invention

The invention relates to the treatment and prevention of symptoms of metabolic syndrome using S100A4.

### Background Art

Decreased insulin sensitivity is usually the first sign of an emerging type 2 diabetes, one of the leading symptoms of metabolic syndrome.

Metformin is the global market leader for oral antidiabetic drugs and the only available antihyperglycaemic agent, which improves both insulin sensitivity and weight loss.

According to the WHO the number of diabetics in 2000 in the WHO Member States was 177 million patients and is estimated at 370 million in 2030. The resulting direct costs will increase from 2.5% to 15% of annual health care expenditures. The recognition that achieving specific glycaemic goals can substantially reduce morbidity, have made the effective treatment of hyperglycemia a top priority. The choice of specific antihyperglycaemic agents is mainly predicated on their blood glucose-lowering effectiveness, extraglycaemic effects that may reduce long-term complications, tolerability, safety profiles, ease of use and expense.

Metformin is the first choice drug for the treatment of type 2 diabetes and the global market leader of oral antidiabetic agents. In Germany alone, it had a turnover of € 51 million. Metformin is believed to act through inhibition of hepatic gluconeogenesis, decreasing intestinal absorption of glucose, increasing the insulin-mediated glucose disposal and inhibition of fatty acid oxidation. Typically metformin monotherapy lowers glucose levels by 20%, and lowers glycosylated hemoglobin levels (HbA1 C) by approximately 1.5 percentage points. Under all available oral antidiabetic agents metformin is the only one, which improves both insulin sensitivity and weight loss. Metformin is very popular because of its low side effects, the prevention of hyperglycaemia, its positive influences on weight and hyperlipidemia, as well as its protective effects against vascular complications. However, besides its gastrointestinal side effects, which remain in 5% of the cases and mostly leads to a discontinuation of the medication, it has a wide range of contraindications. As metformin inhibits the respiratory chain in mitochondria, the main concern is lactic acidosis, which is fatal in 50% of the cases. The risk of lactic acidosis can be minimized by avoiding the well established contraindications for metformin. These contraindications mainly include renal insufficiency, tissue hypoxia as in the case of severe heart and respiratory diseases (e.g. congestive heart failure, coronary artery disease, chronic obstructive pulmonary diseases), hepatic impairment, elderly patients (>80 years), surgical procedures and use of contrast media. 50-60% of the patients treated with metformin have at least one contraindication. As at the moment no comparable alternative is available the contraindications are deemed to be out-weighed by the benefits of the therapy.

S100A4(UniProtKB P26447; 101 amino acids) is a naturally secreted protein and its effect is most likely focused on fat tissue. It is unlikely that it negatively affects the respiratory chain, and it is not contraindicated in liver impairment, heart diseases and kidney insufficiency.

The protein encoded by the S100A4 gene in humans is a member of the S100 family of proteins containing 2 EF-hand calcium-binding motifs. S100 proteins are localized in the cytoplasm and nucleus of a wide range of cells, and involved in the regulation of a number of cellular processes such as cell cycle progression and differentiation.

### Summary of the Invention

The invention relates to the use of S100A4 in the treatment and prevention of symptoms of metabolic syndrome, in particular in the treatment of insulin resistance and type 2 diabetes. This use is preferably indicated in pregnant or breastfeeding women, and in patients with severe heart disease (e.g. heart insufficiency, coronary artery disease), renal insufficiency and/or liver insufficiency.

The invention furthermore relates to pharmaceutical compositions comprising S100A4, and to a method of treatment and prevention of symptoms of metabolic syndrome, in particular a method of treatment of insulin resistance and type 2 diabetes comprising administering to a patient in need thereof an efficient amount of S100A4.

### Brief Description of the Figures

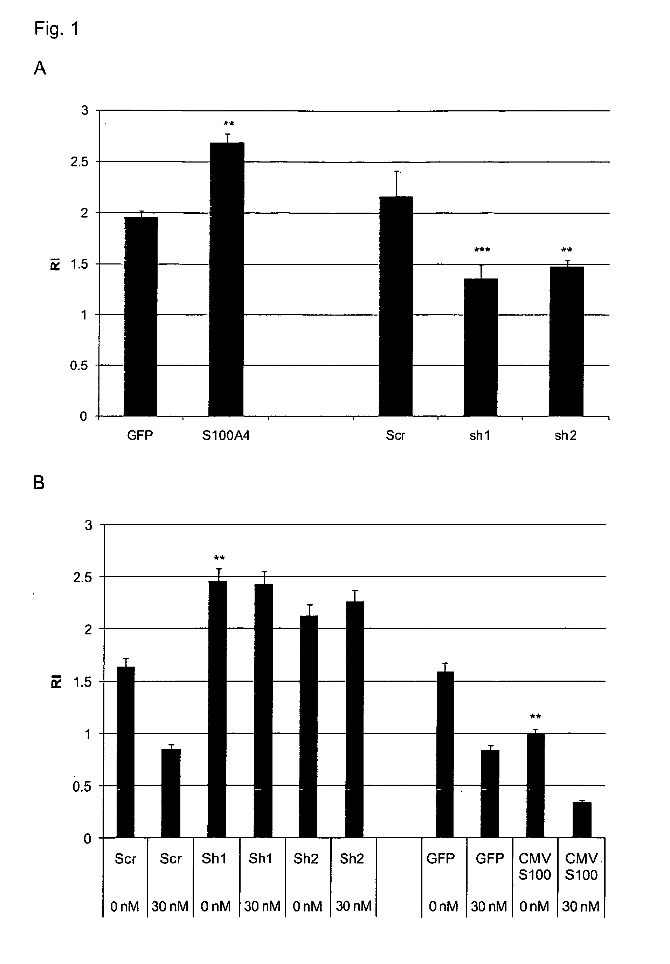

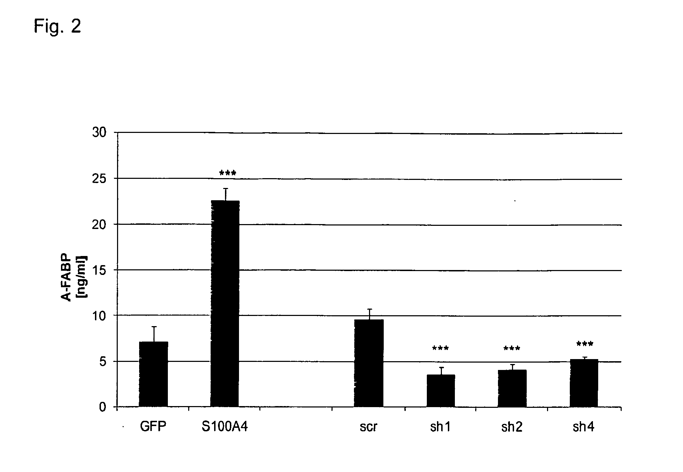

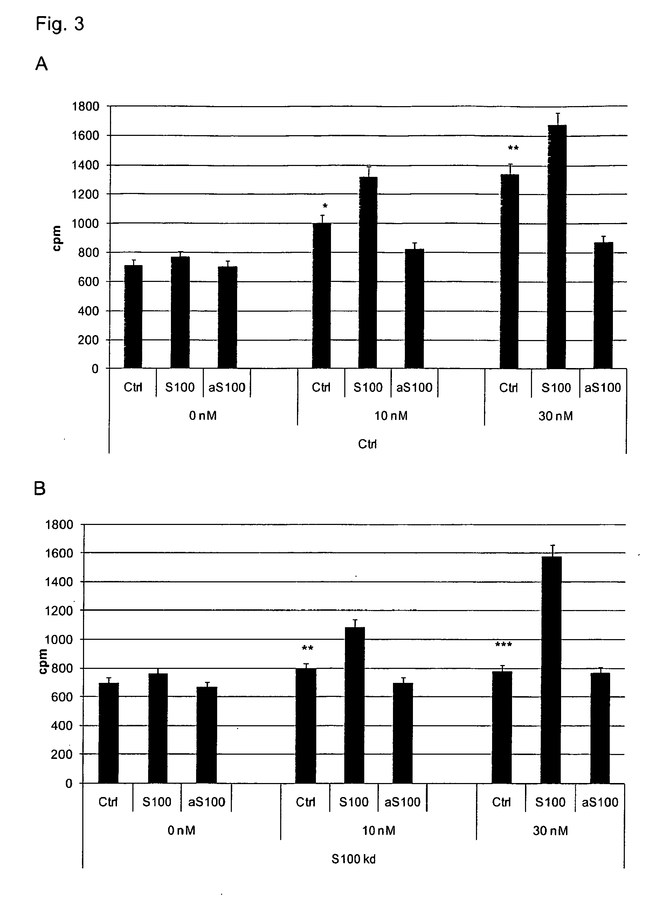

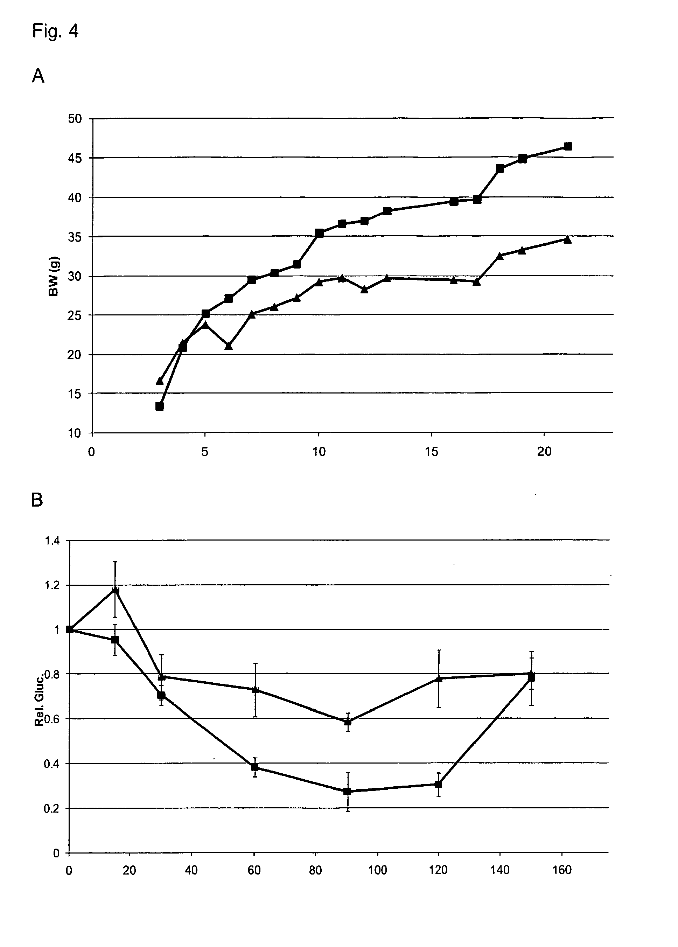

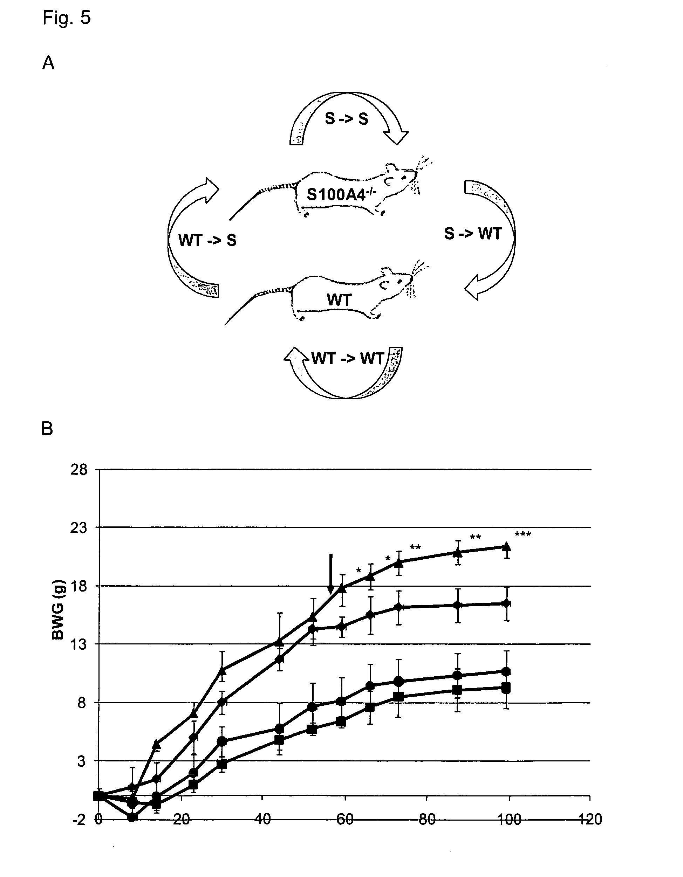

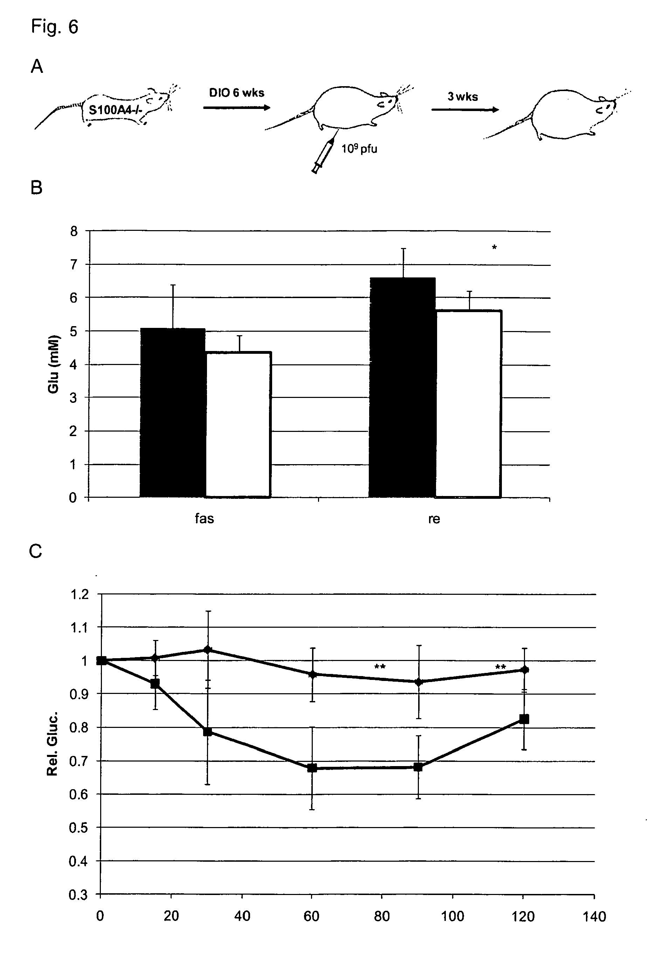

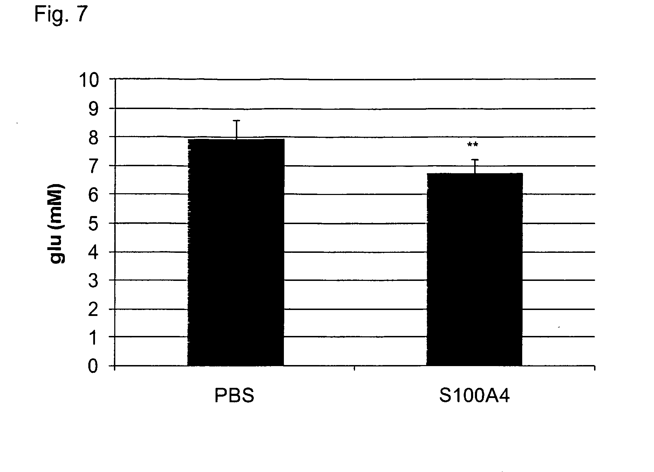

Figure 1: *S100A4 improves insulin sensitivity in adipocytes.*
   Over-expression of S100A4 leads to an increase of insulin sensitivity in adipocytes, whereas a sh-RNA mediated suppression of S100A4 decreases insulin sensitivity. A) Glucose uptake B) Lipolysis
   RI = relative induction; sh = knock-down; S100A4 (or CMV S100) = CMV S100A4 over-expression; GFP = green fluorescent protein; Scr = control for knock down;
   ** = p<0.01; *** = p<0.001
Figure 2: *S100A4 increases adipogenesis.*
   Over-expression of S100A4 leads to an increase of adipogenesis in 3T3L1 preadipocytes, whereas a shRNA mediated suppression of S100A4 decreases adipogenesis. A-FABP = adipocyte fatty acid binding protein; sh = knock-down; S100A4 = CMV S100A4 over-expression; GFP = green fluorescent protein; scr = control for knock down;
   *** = p<0.001
Figure 3: *Recombinant S100A4* increases *adipocyte function*
   Addition of recombinant S100A4 enhances glucose uptake in adipocytes while addition of a S100A4 neutralizing antibody represses glucose uptake. Addition of S100A4 to a cell in which endogenous S100A4 is repressed by knock-down rescues the repressive effect. A) Glucose uptake in normal cells B) Glucose uptake in which S100A4 is reduced by knock out
   Ctrl = Control experiment; S100kd = S100A4 knock down experiment; S100 = addition of S100A4 recombinant protein; aS100 = addition of S100A4 neutralizing antibody; cpm = counts per minute; * = p<0.05; ** = p<0.01; *** = p<0.001
Figure 4: *Increased weight gain and decreased insulin sensitivity in S100A4 knock-out mice.*
   Mice with a null mutation for S100A4 gain more weight than their littermates and show reduced insulin sensitivity in an insulin tolerance test
   A) Body weight B) Insulin tolerance
   BW = Body weight; squares = wild type mice; triangles = S100A4 knock out mice; Rel. Gluc. = relative glucose levels
Figure 5: *Transplantation of* a *fat pad from* a *wild type* mouse *into an* S100A4 *deficient mouse reduces weight gain.*
   Fat pads from mice were transplanted from wild type to wild type, S100A4 knockout to S100A4 knockout, from wild type to S100A4 knockout and from S100A4 knockout to wild type. At 6 weeks after surgery mice were subjected to a high fat diet.
   A) Transplantation scheme B) Body weight gain
   BWG = Body weight gain; triangle = S100A4 knock out to S100A4 knock out mice; rhombus = wildtype to S100A4 knock out mice; circle = wildtype to witdtype mice; square = S100A4 knock out mice to S100A4 knock out mice; arrow = start of high fat diet,
   * = p<0.05, ** = p<0.01, *** = p<0.001
Figure 6: *Elevation of S100A4 improves metabolic parameters in mice, which are on* a *high fat diet.*
   Mice with a null mutation for S100A4 gain more weight than their littermates and show reduced insulin sensitivity in an insulin tolerance test.
   A) Experimental scheme B) Glucose levels C) Insulin tolerance
   Squares = wild type mice; triangles = S100A4 knock out mice; wks = weeks; glu = glucose; fas = fasted; re = refed; rel. gluc. = relative glucose levels;
   * = p<0.05; ** = p<0.01
Figure 7: *Elevation of circulating S100A4 improves glucose homeostasis in mice.*
   Mice are injected with 500 ng/kg S100A4, i.p. and blood glucose levels are monitored. PBS = PBS injected mice; S100A4 = S100A4 injected mice; glu = glucose;
   * = p<0.05; ** = p<0.01

### Detailed Description of the Invention

The scientific rationale for the use of S100A4 in the treatment and prevention of symptoms of metabolic syndrome, in particular in the treatment of insulin resistance and type 2 diabetes, is as follows:

It is demonstrated that S100A4 enhances insulin sensitivity in adipose tissue. This is shown by systemic modulation of S100A4 through viral mediated overexpression or knock down. Over-expression of S100A4 leads to an increase of insulin sensitivity in adipocytes, whereas a shRNA mediated suppression of S100A4 decreases insulin sensitivity (Fig.1). These effects are shown in cell culture models, in which insulin mediated suppression of lipolysis and insulin induced glucose uptake are enhanced by modulation of S100A4 expression. S100A4 also induces adipogenesis by enhancing insulin sensitivity (Fig.2).

It is known that S100A4 is a secreted protein that can be detected in blood plasma. The adipocyte function is analyzed at different extracellular concentrations of S100A4. The addition of recombinant S100A4 protein improves insulin sensitivity as shown by an enhanced insulin stimulated glucose uptake in both, adipocytes with an unmodified S100A4 expression as well as in adipocytes, with a reduced endogenous S100A4 expression (Fig. 3). This shows that the effect of S100A4 is mediated via an extracellular mechanism rather than by an intracellular mechanism.

To investigate the role of S100A4 in mouse model, S100A4 knockout mice and wild type mice are put on a high fat diet, and the impact on energy homoeostasis as well as on emerging metabolic disorders examined. The loss of S100A4 results in a strongly increased weight gain and decreased insulin sensitivity (Fig. 4). An emerging insulin resistance is mainly the first sign for emerging type 2 diabetes.

Since S100A4 acts as a secreted factor, it was examined whether an augmentation of S100A4 blood plasma levels improve the metabolic balance. For this purpose the visceral fat pads of wild type mice (WT) in S100A4 (S) deficient mice are transplanted, and *vice versa.* To control these cross transplants, the visceral fat pads are transplanted within the respective mouse model (Fig. 5). The transplantation of S100A4 secreting visceral fat pads into an S100A4 deficient mouse leads to significant improvement of both, a reduced weight gain as well as increased insulin sensitivity.

To modulate S100A4 expression in a different way adenovirus, causing an overexpression of S100A4, is injected into the visceral fat pads of mice. These mice are on a high fat diet and show the symptoms of metabolic syndrome. The injections are placed directly into the visceral fat pads, to induce an exclusive expression in the fat pad, which should lead to increased circulating S100A4 levels (Fig. 6).

An augmentation of S100A4 improves insulin sensitivity in this model. The effects can already be detected three weeks after the treatment.

To directly assess whether S100A4 can be used to improve insulin signalling and glucose homeostasis WT mice are injected with PBS or S100A4 (i.p.) and blood glucose levels measured. As seen in Fig. 7 a single i.p. injection of S100A4 into a fasted mouse leads to a significant drop in blood glucose levels, demonstrating that circulating protein from an exogenous source can mediate the insulin sensitizing effect.

Taken together it is shown, that the secreted protein S100A4 significantly improves the insulin sensitivity of the organism. This decelerates or suppresses the development of metabolic disorders such as obesity and type 2 diabetes. For this reason S100A4 is a new therapeutic approach for the treatment of the symptoms of metabolic syndrome, such as type 2 diabetes and obesity. Administration of S100A4 in a mouse model also leads to an improvement of insulin sensitivity. Furthermore, S100A4 knock out mice show a strongly increased weight gain on high fat diet in comparison to wild type mice. The transplantation of a visceral wild type fat pad into a S100A4 knock out mice decreased weight gain and led to an improvement of insulin sensitivity. Additionally, the selective over-expression of S100A4 in the visceral fat pads of mice, which are on a high fat diet and show the symptoms of metabolic syndrome, significantly increases the insulin sensitivity of those mice.

Augmentation of S100A4 blood levels leads to a systemic improvement of insulin sensitivity and to an improved control of weight gain. Therefore, S100A4 is a new therapy approach for the treatment of type 2 diabetes and metabolic syndrome. S100A4 also increases insulin sensitivity and is a new therapeutic approach especially for the increasing number of patients, for which metformin and other current antidiabetic agents are contraindicated.

S100A4 is particularly suitable for application in case of side effects and contraindications of metformin. Metformin is contraindicated in severe liver disease and renal insufficiency, which occurs frequently in older patients. It is also contraindicated in slimming diets, alcoholism and diseases associated with poor oxygen supply to the body (heart failure or severe respiratory insufficiency), after a fresh heart attack and in elderly patients (>80 years). Additionally, pregnancy or breastfeeding are also exclusion criteria for metformin.

Besides Metformin, sulfonylurea is another well-validated medication and frequently used in diabetes therapy. The disadvantages of this drug are hypoglycaemia and weight gain. Similar to the profile of metformin it is also contraindicated in severe liver disease, pregnancy and breastfeeding and very problematic in use for patients with renal insufficiency. The frequently occurring diseases of heart insufficiency (NYHAI-IV) and liver impairment are also contraindications for the PPARy ligands and pioglitazone, another popular group of antidiabetic drugs. Taken together, frequently occurring diseases as renal insufficiency and liver impairment, as well as breastfeeding are contraindications for the main oral antihyperglycaemic agents. In those cases S100A4 is an interesting alternative. Furthermore S100A4 is particularly suitable in pregnancy, a general contraindication of oral antidiabetics.

### Pregnancy in diabetic women

Diabetes occurs in 0.3-0.5% of all pregnancies in Europe and the USA. The fetus cannot produce insulin up to the 12**^{th}** week of pregnancy. A hyperglycaemia of the mother thus also leads to a hyperglycaemia of the fetus. Poor blood sugar control triples the risk of malformation up to 6-10%.

### Gestational diabetes

Gestational diabetes is an emerging type 2 diabetes during pregnancy, which occurs in 1-3% of all pregnancies in Europe and the USA. The cause is decreased insulin sensitivity, as well as an increased secretion of insulin antagonists such as cortisol, estrogen, human placental lactogen and prolactin.

### S100A4 therapy in pregnancy

The available oral antidiabetic agents cross the placenta, and teratogenic effects cannot be excluded. For this reason, the use of insulin is presently the therapy of choice during pregnancy. Under this therapy frequently occurring hypoglycaemia or sporadically occurring hyperglycaemia of the mother and the child must be accepted.

S 1 00A4 does not pass the placental barrier. Due to its insulin sensitivity-enhancing effect and the avoidance of hypoglycaemia, S1 00A4 is the preferred treatment of diabetes in pregnant woman.

A pharmaceutical composition according to the invention comprises S100A4 and a pharmaceutically acceptable carrier, excipient or stabilizer. Suitable carriers, excipients or stabilizers known to the skilled person in the art are saline, Ringer's solution, dextrose solution, Hank's solution, fixed oils, ethyl oleate, 5% dextrose in saline, substances that enhance isotonicity and chemical stability, buffers and preservatives. Other suitable carriers include further proteins, polysaccharides, polylactic acids, polyglycolic acids, polymeric amino acids and amino acid copolymers. A pharmaceutical composition may also be a combination formulation, comprising an additional active agent, such as one of the anti-diabetic compounds of the state of the art.

The formulations to be used for *in vivo* administration must be aseptic or sterile. This is readily accomplished by filtration through sterile filtration membranes.

Sustained-release preparations may be prepared. In one embodiment of the invention, an implant can be used for providing S100A4 according to the invention. Suitable examples of sustained-release preparations include semipermeable matrices of solid hydrophobic polymers containing S100A4, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides, copolymers of L-glutamic acid and gamma-ethyl-L-glutamate, non-degradable ethylenevinyl acetate, degradable lactic acid-glycolic acid copolymers such as injectable microspheres composed of lactic acid-glycolic acid copolymer and leuprolide acetate, and poly-D-(-)-3-hydroxybutyric acid.

The pharmaceutical composition may be administered by any suitable method within the knowledge of the skilled person. The preferred route of administration is parenterally. In parental administration, S100A4 will be formulated in a unit dosage injectable form such as a solution, suspension or emulsion, in association with the pharmaceutically acceptable excipients as defined above. The dosage and mode of administration will depend on the individual to be treated and the particular disease. Generally, the pharmaceutical composition is administered so that S100A4 is given at a dose between 0.1 and 100 µg/kg, more preferably between 0.2 and 10 µg/kg, most preferably between 0.5 and 1 µg/kg. Preferably, it is given as a bolus dose. Continuous infusion may also be used and includes continuous subcutaneous delivery *via* an osmotic minipump. If so, the pharmaceutical composition may be infused at a dose between 0.01 and 1 µg/kg/minute, more preferably between 0.05 and 0.1 µg/kg/minute.

### Examples

### Animals

All mouse models were maintained in the C57BI/6J background and kept on a 12 h/12 h light/dark cycle in a pathogen-free animal facility. Groups of mice were fed a high-fat diet (Provimi Kliba AG) containing 60% fat for 8 weeks. O₂, CO₂, food and water intake were simultaneously determined for four mice per experiment in an Oxymax metabolic cage system (Columbus Instruments).

### Cell culture

3T3-L1 preadipocytes were cultured on collagen-coated plates in high-glucose Dulbecco's modified Eagle's medium (Invitrogen) supplemented with 10% fetal bovine serum and penicillin/streptomycin. Adipogenesis was induced by treating postconfluent 3T3-L1 preadipocytes or 80% confluent stromal-vascular cells with insulin, dexamethasone, and isobutylmethylxanthine.

### Lentiviral infection

Lentiviral stocks were prepared the following way. HEK293T cells were transfected using lipofectamine with lentiviral overexpression or control vector and the packaging vectors pMD2.G and psPAX2. Virus containing medium was collected 24 h after changing transfected cells to high BSA (1.1 g / 100 ml) D-MEM supplemented with 10% fetal bovine serum and penicillin/streptomycin. For functional assays of 3T3-L1 adipocytes, 3T3-L1 cells were infected for 24 h with virus containing medium supplemented with polybrene (8 µg/ml) two days after induction of differentiation. Differentiation was continued until day 8.

### Glucose uptake and lipolysis

Adipocytes were preincubated with 20 nM (primary adipocytes) or 50 nM (3T3-L1 adipocytes) insulin for 10 min. Cells were incubated for 1 h with 1 mM glucose spiked with D-[1-¹⁴C]glucose in Krebs-Ringer buffer adding 1% BSA for primary adipocytes. Supernatant of 3T3-L1 adipocytes was collected, cells were washed, lysed and ¹⁴C incorporation as well as total protein content by BCA Assay (Sigma) was measured. Primary adipocytes were spun through dinonyl phthalate oil, washed and the aqueous phase was collected. Osmium tetroxide fixed primary adipocytes were counted for normalization using a Coulter Cell Counter. Glycerol release in the supernatant was measured to assess lipolysis using glycerol reagent (Sigma) according to the manufacturer's protocol.

### In vivo differentiation of SVF

Fat tissue was dissected, minced and incubated in collagenase type II for 1 h at 37°C. Pelleted stomal-vascular fraction was strained through a 40 µm net, erythrocytes were lysed and 10⁶ cells were resuspended in 100 µl of Matrigel [BD]. Cells were injected subcutaneously into a skin fold underneath the neck. After 4-6 weeks Matrigel pads were excised and treated as described below for the determination of adipocyte size. From each pad pictures of 3 full sections were taken and adipocyte numbers as well as the number of nuclei were determined automatically with Cell Profiler Software.

### Generation of recombinat S100A4

S100A4 was purified twice from E.coli via the His-tag using a Ni-NTA column (GE Healthcare) connected to an automated FPLC system. S100A4 was dialyzed with Slide-A-Lyzer® Dialysis Cassettes (Thermo Scientific) against PBS and analyzed by Western Blot using anti-His tag antibody (Cell Signalling) or Coomassie-stained 12% SDS-polyacrylamide gel.

### Fat pad transplantation

Six-week-old male wild type mice (WT) and S100A4 deficient mice (S) were used as recipients and donors of visceral fat pads. The visceral fat pads of wild type mice were transplanted in S100A4 deficient mice, and *vice versa.* To control these cross transplants, the visceral fat pads were transplanted within the respective mouse model. Fat transplantation was performed using fat pads removed from the intra-abdominal perigonadal (epididymal) area. The epididymal fat pads were transplanted in the intra-abdominal cavity and fixed with 2 stitches at the peritoneum close to their physiological position. Analgesia was performed pre-operatively with buprenorphine (0.05 mg/kg i.p.) and post-operatively by adding codein (0.024 mg/ml) and para-(acetylamino)phenol (0.24 mg/ml) in the drinking water for three days. For antibiosis enrofloxacin 10% (1.44 µg/ml) was added post-operatively to the drinking water for three days. After the surgery the animals were maintained on a high fat diet and a standard 12 h light/dark cycle.

### Adenoviral injection into fat pad

Nine-week-old wild type mice were on a high fat diet for three weeks when S1 00A4 adenovirus (1.1 x 10¹² pts), causing an overexpression of S100A4, was injected in the visceral fat pads. To place the injections exclusively into the epididymal fat pad, the intra-abdominal cavity was opened and the epididymal fat pads were exposed. As a control GFP adenovirus (1.1 x 10¹² pts) was injected in another cohort. Analgesia was performed pre-operatively with buprenorphine (0.05 mg/kg i.p.) and post-operatively by adding codein (0.024 mg/ml) and para-(acetylamino)phenol (0.24 mg/ml) in the drinking water for three days. For antibiosis enrofloxacin 10% (1.44 µg/ml) was added to the drinking water for three days. After the surgery the animals were maintained on a high fat diet and a standard 12 h light/dark cycle.

### S100A4 i.p. injection

S100A4 was injected at 500 ng/kg body weight i.p. into mice that were fed a high fat diet for 3 weeks prior to the start of the experiment. After 2 h animals were killed and blood parameters measured.

### Automated analysis of adipocyte differentiation

Differentiated cells were fixed with 5% formaldehyde prior to staining with BODIPY for lipid droplets, Hoechst for nuclei and Syto60 for cytosolic staining (all Invitrogen). 25 pictures per well were taken with an automated microscope imaging system (CellWorx). Pictures were analyzed using Cell Profiler Software.

### Serum measurements

Blood glucose was measured with a glycometer (Contour). Insulin was determined with the Rat/Mouse Insulin ELISA kit (Crystal Chem).

### Statistical analysis

Statistical analyses were performed using a two-tailed Student's t test.

## Claims

1. S100A4 for use in the treatment and prevention of symptoms of metabolic syndrome.

2. S100A4 for use in the treatment of insulin resistance and type 2 diabetes according to claim 1.

3. S100A4 for use in the treatment of insulin resistance and type 2 diabetes according to claim 1 of pregnant or breastfeeding women.

4. S100A4 for use in the treatment of gestational diabetes according to claim 3.

5. S100A4 for use in the treatment of insulin resistance and type 2 diabetes according to claim 1 of patients with severe heart disease, renal insufficiency and/or liver insufficiency.

6. S100A4 for use in the treatment of insulin resistance and type 2 diabetes according to claim 1 of elderly patients above 80 years of age.

7. A pharmaceutical composition comprising S100A4.

8. A method of treatment and prevention of symptoms of metabolic syndrome comprising administering to a patient in need thereof an efficient amount of S100A4.

9. The method of treatment of insulin resistance and type 2 diabetes according to claim 6 comprising administering to a patient in need thereof an efficient amount of S100A4.
